# EUROPÄISCHE PATENTSCHRIFT

(11) **EP 0 819 679 B1**
(45) Veröffentlichungstag und Bekanntmachung des Hinweises auf die Patenterteilung: **06.11.2002**
(21) Anmeldenummer: 97111346.9
(22) Anmeldetag: 04.07.1997
(51) Int. Cl.: C07D 239/52, C07D 239/28, C07D 239/34, C07D 239/60

(54) **Verfahren zur Herstellung von Pyrimidin-2-carbonsäureestern**
Process for the preparation of pyrimidine-2-carboxylic acid esters
Procédé pour la préparation d'esters d'acide pyrimidine-2-carboxylique

(30) Priorität: 18.07.1996 CH 179796
(43) Veröffentlichungstag der Anmeldung: 21.01.1998
(73) Patentinhaber: Lonza AG, 3930 Visp (CH)
(72) Erfinder: Bessard, Yves, Dr., 3960 Sierre (Kanton Wallis) (CH); Stucky, Gerhard, Dr., 3902 Brig-Glis (Kanton Wallis) (CH)
(74) Vertreter: Winter, Brandl, Fürniss, Hübner, Röss, Kaiser, Polte Partnerschaft

(56) Entgegenhaltungen:
- EP-A- 0 282 266
- EP-A- 0 582 288
- DE-A- 3 826 230
- CHEMICAL ABSTRACTS, vol. 125, no. 3, 15.Juli 1996 Columbus, Ohio, US; abstract no. 33323, XP002047592 & JP 08 073 403 A (NIHON NOHYAKU CO LTD) 19.März 1996
- H. EILINGSFELD ET AL: CHEM. BER., Bd. 101, 1968, Seiten 2426-2434, XP002047590
- H. NEUNHOEFFER ET AL: LIEBIGS ANN. CHEM., Nr. 8, 1974, Seiten 1190-1194, XP002047591

## Beschreibung

Die vorliegende Erfindung betrifft ein Verfahren zur Herstellung von substituierten Pyrimidin-2-carbonsäureestern durch Umsetzung von 2-Halopyrimidinen mit Kohlenmonoxid und einem Alkohol in Gegenwart eines Katalysators und einer Base.

Die erfindungsgemäss herstellbaren Ester besitzen die allgemeine Formel

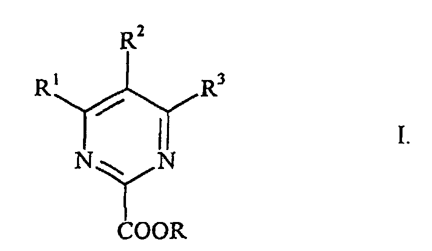

Hierin bedeuten:
R C₁₋₆-Alkyl, C₃₋₆-Cycloalkyl, oder gegebenenfalls mit C₁₋₆-Alkyl, halogeniert oder unhalogeniert, C₁₋₆-Alkoxy, C₁₋₆-Alkylthio, C₁₋₆-Alkansulfonyl oder Fluor substituiertes Aryl oder Aryl-C₁₋₆-alkyl,
R¹ bis R³ unabhängig voneinander Wasserstoff, C₁₋₆-Alkyl, fluoriertes C₁₋₆-Alkyl, C₁₋₆-Alkoxy, (C₁₋₆-Alkoxy)-C₁₋₆-alkyl oder (C₁₋₆-Alkoxy)-carbonyl.

Verbindungen dieser Struktur sind Zwischenprodukte für die Herstellung von Herbiziden (EP-A 0 152 286, DE-A 38 26 230) oder pharmazeutischen Wirkstoffen (DE-A 23 41 925). Bekannte Synthesen dieser Verbindungen gehen beispielsweise von den entsprechenden 2-Methylpyrimidinen aus, deren Methylgruppe mit Kaliumpermanganat zur Carboxylgruppe oxidiert und anschliessend verestert wird (siehe beispielsweise H. Neunhoeffer und G. Werner, *Liebigs Ann. Chem.* **1974**, 1190-1194). Die Synthese der Verbindungen mit R¹ = R³ = Alkoxy geht üblicherweise von den aus Malonsäuredinitril und Alkoholen erhältlichen Propandiimidsäureestern und den Oxalsäuremonoesterchloriden aus (H. Eilingsfeld et al., *Chem. Ber*. **1968,** *101,* 242-2434). Ein Verfahren zur Herstellung von heteroaromatischen Carbonsäureestern, beispielsweise von Nikotinsäureestern, wird in der EP-A-0 282 266 beschrieben, wobei chlorsubstituierte Pyridine mit Kohlenmonoxid und einem Amin oder einem Alkohol in Gegenwart einer Base mit einem Palladium-Phosphin-Komplex umgesetzt werden.

Aufgabe der vorliegenden Erfindung war, ein alternatives Verfahren zur Herstellung von substituierten Pyrimidin-2-carbonsäureestern zur Verfügung zu stellen, welches von leicht zugänglichen Edukten ausgeht und hohe Ausbeuten liefert.

Erfindungsgemäss wird diese Aufgabe durch das Verfahren nach Patentanspruch 1 gelöst. Es wurde gefunden, dass 2-Halopyrimidine der allgemeinen Formel 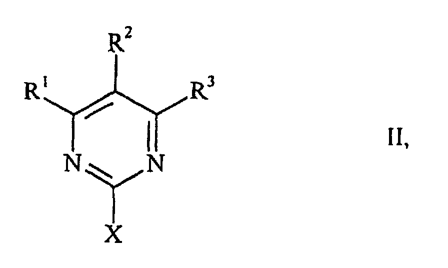 worin R¹ bis R³ die oben genannten Bedeutungen haben und X Chlor oder Brom ist, mit Kohlenmonoxid und einem Alkohol der allgemeinen Formel

R-OH III,

worin R die oben genannte Bedeutung hat, in Gegenwart einer Base in guter Ausbeute direkt zu den gewünschten Produkten (I) reagieren, wenn als Katalysator ein Palladium-Phosphin-Komplex eingesetzt wird.

Unter C₁₋₆-Alkyl sind hier und im folgenden alle linearen oder verzweigten primären, sekundären oder tertiären Alkylgruppen mit bis zu 6 Kohlenstoffatomen zu verstehen. Entsprechend sind unter C₁₋₆-Alkoxy und (C₁₋₆-Alkoxy)carbonyl die aus C₁₋₆-Alkyl und Sauerstoff bzw. Sauerstoff und Carbonyl zusammengesetzten Ether- und Esterfunktionen zu verstehen und analog dazu unter (C₁₋₆-Alkoxy)-C₁₋₆-alkyl die durch Austausch eines Wasserstoffatoms in C₁₋₆-Alkyl gegen C₁₋₆-Alkoxy gebildeten Alkoxyalkylgruppen, also beispielsweise Methoxymethyl oder Ethoxymethyl.
Unter Aryl sind hier und im folgenden insbesondere mono- oder polycyclische Systeme wie beispielsweise Phenyl, Naphthyl, Biphenylyl oder Anthracenyl zu verstehen. Diese können einen oder mehrere gleiche oder verschiedene Substituenten tragen, nämlich C₁₋₆-Alkylgruppen wie Methyl, halogenierte C₁₋₆-Alkylgruppen wie Trifluormethyl, C₁₋₆-Alkoxygruppen wie Methoxy, oder C₁₋₆-Alkylthio- (Alkansulfanyl-) oder Alkansulfönylgruppen wie Methylthio oder Ethansulfonyl. Unter substituiertem Phenyl sind insbesondere Gruppen wie Fluorphenyl, Methoxyphenyl, Tolyl oder Trifluormethylphenyl zu verstehen, wobei sich die Substituenten vorzugsweise in *para*-Stellung befinden. Entsprechend sind unter Aryl-C₁₋₆-alkyl die aus C₁₋₆-Alkylgruppen durch Austausch eines Wasserstoffatoms gegen eine der vorstehend definierten Arylgruppen gebildeten Gruppen zu verstehen, also beispielsweise Benzyl oder Phenylethyl.

Die als Ausgangsmaterial dienenden 2-Halopyrimidine (II) sind bekannte Verbindungen oder können analog zu bekannten Verbindungen hergestellt werden. Ein Verfahren zur Herstellung von 2-Halo-4,6-dialkoxypyrimidinen ist beispielsweise in EP-A 0 582 288 beschrieben.
Vorzugsweise werden als 2-Halopyrimidine die 2-Chlorpyrimidine (X = Cl) eingesetzt.

Vorzugsweise werden nach dem erfindungsgemässen Verfahren C₁₋₄-Alkylester hergestellt (R = C₁₋₄-Alkyl), indem als Alkohol (III) das entsprechende C₁₋₄-Alkanol eingesetzt wird. Besonders bevorzugt sind Methyl-, Ethyl- und Isopropylester.

Ebenfalls bevorzugt ist die Herstellung von Pyrimidin-2-carbonsäureestem (I), die in der Position 5 des Pyrimidinrings unsubstituiert sind (R² = H).

Besonders bevorzugt ist die Herstellung von Pyrimidin-2-carbonsäureestern (I), die in den Positionen 4 und 6 des Pyridinrings (R¹, R³) Wasserstoff, C₁₋₄-Alkoxygruppen, (C₁₋₄-Alkoxy)carbonylgruppen oder (C₁₋₄-Alkoxy)methylgruppen tragen.

Als Phosphin im katalytisch aktiven Palladium-Phosphin-Komplex wird vorteilhaft ein tertiäres Phosphin eingesetzt. Geeignet sind beispielsweise Triarylphosphine wie Triphenylphosphin oder an den Phenylgruppen substituierte Triphenylphosphine oder Diarylphosphine, bei denen die dritte Valenz am Phosphor durch einen anderen organischen Rest, beispielsweise eine aliphatische Kette oder ein Metallocenylsystem, besetzt ist. Vorzugsweise eingesetzt werden Diphosphine der allgemeinen Formel

R⁴R⁵P―Q―PR⁶R⁷ IV,

worin R⁴ bis R⁷ unabhängig voneinander gegebenenfalls substituiertes Phenyl, C₁₋₆-Alkyl oder C₃₋₆-Cycloalkyl bedeuten und Q eine 1,1'-Ferrocendiylgruppe oder eine Gruppe der Formel -[CH₂]_{*n*}-, worin *n* 3 oder 4 ist, bedeutet.

Der katalytisch wirksame Palladium-Phosphin-Komplex wird vorteilhaft *in situ* gebildet, indem Palladium in fein verteilter elementarer Form (z. B. Palladium auf Aktivkohle), ein Pd(II) Salz (z.B. das Chlorid oder das Acetat), oder ein geeigneter Pd(II)-Komplex (z. B. Dichloro-bis(triphenylphosphin)palladium(II)) mit dem Phosphin zur Reaktion gebracht wird. Besonders bevorzugt sind Palladium(II)acetat und Dichloro-bis(triphenylphosphin)-palladium(II). Das Palladium wird vorzugsweise in einer Menge von 0,02 bis 2 Mol-% Pd(II) oder 0,5 bis 5 Mol-% Pd(0) (als Pd/C), jeweils bezogen auf die Halogenverbindung (II), eingesetzt. Das Phosphin wird vorteilhaft im Überschuss (bezogen auf Pd) eingesetzt, vorzugsweise in einer Menge von 0,2 bis 10 Mol-%, ebenfalls bezogen auf die Halogenverbindung (II).

Der Alkohol (III) kann auch gleichzeitig als Lösungsmittel dienen. Gegebenenfalls kann ein zusätzliches Lösungsmittel eingesetzt werden. Als zusätzliche Lösungsmittel kommen sowohl relativ unpolare, wie beispielsweise Toluol oder Xylol, als auch polare wie beispielsweise Acetonitril, Tetrahydrofuran oder *N,N*-Dimethylacetamid in Frage.

Als Base wird vorzugsweise eine schwache Base aus der Gruppe der Alkali- oder Erdalkalisalze von niedrigen Carbonsäuren, der Alkali- oder Erdalkalihydrogencarbonate oder der Alkali- oder Erdalkali(di)hydrogenphosphate eingesetzt. Besonders bevorzugt sind Alkaliacetate, insbesondere Natrium- und Kaliumacetat.

Die Reaktionstemperatur liegt vorzugsweise bei 80 bis 250 °C.

Der Kohlenmonoxiddruck liegt vorzugsweise bei 1 bis 50 bar.

Die Reaktionsdauer hängt unter anderem von der Temperatur, der Reaktivität der eingesetzten Verbindungen und den Konzentrationsverhältnissen ab, sie liegt typischerweise im Bereich von einigen Stunden. Da bei übermässig langer Reaktionsdauer unter Umständen Folgereaktionen eintreten, wird der Reaktionsverlauf vorteilhaft mit einer geeigneten Analysenmethode (z. B. GC) überwacht und die Reaktion nach Erreichen der maximalen Produktkonzentration abgebrochen.

Die folgenden Beispiele verdeutlichen die Durchführung des erfindungsgemässen Verfahrens.

### Beispiel 1

### 4,6-Dimethoxypyrünidin-2-carbonsäuremethylester (I, R = Me, R¹ = R³ = OMe, R² = H)

In einem indirekt beheizten (Ölbad) Metallautoklaven wurden 3,49 g (20 mmol) 2-Chlor-4,6-dimethoxypyrimidin (hergestellt nach EP-A- 0 582 288), 256 mg (0,6 mmol) 1,4-Bis-(diphenylphosphino)butan, 28 mg (40 µmol) Dichlorobis(triphenylphosphin)palladium(II), 4,92 g (60 mmol) Natriumacetat, 1,92 g (60 mmol) Methanol und 56 ml Tetrahydrofuran vorgelegt. Der Autoklav wurde mehrmals mit Kohlenmonoxid gespült, dann wurde der Kohlenmonoxiddruck auf 15 bar erhöht und das Reaktionsgemisch 6 h bei 180 °C Badtemperatur erhitzt. Eine GC-Analyse des Reaktionsgemischs ergab eine Ausbeute von 99% bei einem Umsatz von 100%. Zur Aufarbeitung wurde das Reaktionsgemisch im Vakuum eingeengt und der Rückstand an Kieselgel 60 mit Hexan/Ethylacetat (1:1) chromatographiert.
Isolierte Ausbeute: 1,0 g (71%) farblose Kristalle
Schmp.: 129,7-131,1 °C
¹H NMR (CDCl₃)δ = 6,15 (s, 1H); 4,03 (s, 6H); 4,00 (s, 3H).
MS (*m*/*z*): 198 (M⁺); 197; 183; 168; 139; 125; 108; 93.

### Beispiel 2

### 4,6-Dimethoxypyrimidin-2-carbonsäuremethylester (I, R = Me, R¹ = R³ = OMe, R² = H)

Es wurde verfahren wie in Beispiel 1 beschrieben, jedoch wurde anstelle von Dichlorobis-(triphenylphosphin)palladium(II) 9,0 mg (40 µmol) Palladium(II)acetat und anstelle von 1,4-Bis(diphenylphosphino)butan 333 mg (0,6 mmol) 1,1'-Bis(diphenylphosphino)ferrocen eingesetzt. Die Badtemperatur betrug 165 °C, die Reaktionsdauer 4 h. Eine GC-Analyse des Reaktionsgemischs ergab eine Ausbeute von 92% bei gleich grossem Umsatz.
Isolierte Ausbeute: 3,36 g (85%) farblose Kristalle

### Beispiel 3

### 4,6-Dimethoxypyrimidin-2-carbonsäuremethylester (I, R = Me, R¹ = R³ = OMe, R² = H)

Es wurde verfahren wie in Beispiel 2 beschrieben, jedoch wurde das Tetrahydrofuran durch Methanol ersetzt (Gesamtmenge: 50 ml) und die Reaktionsdauer auf 2 h reduziert. Eine GC-Analyse des Reaktionsgemischs zeigte quantitative Ausbeute und Umsatz an.
Isolierte Ausbeute: 3,54 g (90%) hellbeige Kristalle

### Beispiel 4

### 4,6-Dimethoxypyrimidin-2-carbonsäuremethylester (I, R = Me, R¹ = R³ = OMe, R² = H)

Es wurde verfahren wie in Beispiel 3 beschrieben, jedoch wurde das 1,1'-Bis(diphenylphosphino)ferrocen durch 163 mg (0,6 mmol) Triphenylphosphin ersetzt. Die Reaktionsdauer betrug 4 h bei 170 °C Badtemperatur. Eine GC-Analyse des Reaktionsgemischs zeigte eine Ausbeute von 42% (neben 58% unumgesetztem Ausgangsmaterial) an.

### Beispiel 5

### 4,6-Dimethoxypyrimidin-2-carbonsäuremethylester (I, R = Me, R¹ = R³ = OMe, R² = H)

Es wurde verfahren wie in Beispiel 4 beschrieben, jedoch wurde das Triphenylphosphin durch 211 mg (0,6 mmol) Tris(4-methoxyphenyl)phosphin ersetzt. Eine GC-Analyse des Reaktionsgemischs zeigte eine Ausbeute von 62% (neben 38% unumgesetztem Ausgangsmaterial) an.

### Beispiel 6

### 4,6-Dimethoxypyrimidiu-2-carbousäureethylester (I, R= Et, R¹ = R³ = OMe, R² = H)

Es wurde verfahren wie in Beispiel 3 beschrieben, jedoch wurde anstelle von Methanol 50 ml Ethanol eingesetzt. Die Reaktionsdauer betrug *2,5* h bei 160 °C Badtemperatur. Eine GC-Analyse des Reaktionsgemischs zeigte eine Ausbeute von 95,5% (neben 4,5% unumgesetztem Ausgangsmaterial) an.
Isolierte Ausbeute: 3,71 g (86,7%) blassgelbe Kristalle, Gehalt (GC) 99,2%
Schmp.: 55,9-57,4 °C
¹HNMR (CDCl₃) δ = 6,15 (s, 1H); 4,46 (q, 2H); 4,02 (s, 6H); 1,43 (t, 3H).
MS(*m*/*z*): 212(M⁺); 211; 183; 154; 140; 125.

### Beispiel 7

### 4,6-Dimethoxypyrimidin-2-carbonsäureisopropylester (I, R = i-Pr, R¹ = R³ = OMe, R² = H)

Es wurde verfahren wie in Beispiel 3 beschrieben, jedoch wurde anstelle von Methanol 50 ml Isopropylalkohol eingesetzt. Die Reaktionsdauer betrug 4 h bei 170 °C Badtemperatur. Eine GC-Analyse des Reaktionsgemischs zeigte eine Ausbeute von 87% bei einem Umsatz von 92% an.
Isolierte Ausbeute: 2,46 g (54%) grünliche Kristalle, Gehalt (GC) 100%
Schmp.: 64,6-66,6 °C
¹H NMR (CDCl₃) δ = 6,15 (s, 1H); 5,28 (sept., 1H); 4,01 (s, 6H); 1,42 (d, 6H).
MS (*m*/*z*): 226 (M⁺); 211; 183; 167; 140.

### Beispiel 8

### 4,6-Dimethoxypyrimidin-2-carbonsäurecyclohexylester (I, R = Cyclohexyl, R¹ = R³ = OMe, R² = H)

Es wurde verfahren wie in Beispiel 3 beschrieben, jedoch wurde das Methanol durch 50 ml Cyclohexanol ersetzt. Die Reaktionsdauer betrug 3 h, die Badtemperatur 160 °C.
Isolierte Ausbeute: 4,08 g (75%) hellgelbe Kristalle
Schmp.: 99,0-101,2 °C
¹H NMR (CDCl₃) δ = 6,15 (s, 1H); 5,07 (m, 1H); 4,01 (s, 6H); 2,0 (m, 2H); 1,8 (m, 2H); 1,6 (m, 3H); 1,4 (m, 3H).
MS (*m*/*z*): 266 (M⁺); 221; 185; 167; 139.

### Beispiel 9

### 4,6-Dimethoxypyrimidin-2-carbonsäurebenzylester (I, R = Benzyl, R¹ = R³ = OMe, R² = H)

Es wurde verfahren wie in Beispiel 3 beschrieben, jedoch wurde das Methanol durch 4,33 g Benzylalkohol und 50 ml Tetrahydrofuran ersetzt. Die Reaktionsdauer betrug 3 h, die Badtemperatur 160 °C.
Isolierte Ausbeute: 4,18 g (76%) beige Kristalle
Schmp.: 96,8-98,1 °C
¹H NMR (CDCl₃) δ = 7,50 ("d", 2H); 7,45 (m, 3H); 6,15 (s, 1H); 5,43 (s, 2H); 4,01 (s, 6H).
MS (*m*/*z*): 274 (M⁺); 246; 168; 140.

## Patentansprüche

1. Verfahren zur Herstellung von Pyrimidin-2-carbonsäureestern der allgemeinen Formel 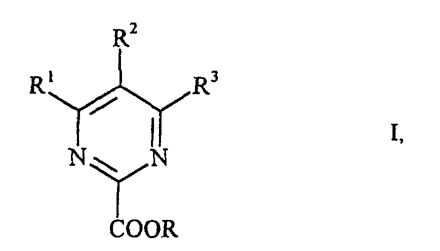 worin R C₁₋₆-Alkyl, C₃₋₆-Cycloalkyl, oder gegebenenfalls mit C₁₋₆-Alkyl, halogeniert oder unhalogeniert, C₁₋₆-Alkoxy, C₁₋₆-Alkylthio, C₁₋₆-Alkansulfonyl oder Fluor substituiertes Aryl oder Aryl-C₁₋₆-alkyl ist und R¹ bis R³ unabhängig voneinander Wasserstoff, C₁₋₆-Alkyl, fluoriertes C₁₋₆-Alkyl, C₁₋₆-Alkoxy, (C₁₋₆-Alkoxy)-C₁₋₆-alkyl oder (C₁₋₆-Alkoxy)carbonyl bedeuten, **dadurch gekennzeichnet, dass** ein 2-Halopyrimidin der allgemeinen Formel 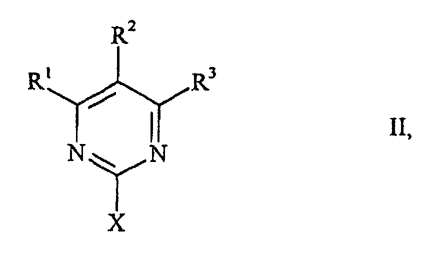 worin R¹ bis R³ die oben genannten Bedeutungen haben und X Chlor oder Brom ist, mit Kohlenmonoxid und einem Alkohol der allgemeinen Formel
R-OH III,
worin R die oben genannte Bedeutung hat, in Gegenwart eines katalytisch aktiven Palladium-Phosphin-Komplexes und einer Base zur Reaktion gebracht wird.

2. Verfahren nach Anspruch 1, **dadurch gekennzeichnet, dass** X Chlor ist.

3. Verfahren nach Anspruch 1 oder 2, **dadurch gekennzeichnet, dass** R C₁₋₄-Alkyl ist.

4. Verfahren nach einem der Ansprüche 1 bis 3, **dadurch gekennzeichnet, dass** R² Wasserstoff ist.

5. Verfahren nach einem der Ansprüche 1 bis 4, **dadurch gekennzeichnet, dass** R¹ und R³ unabhängig voneinander Wasserstoff, C₁₋₄-Alkoxy, (C₁₋₄-Alkoxy)carbonyl, oder (C₁₋₄-Alkoxy)methyl sind.

6. Verfahren nach einem der Ansprüche 1 bis 5, **dadurch gekennzeichnet, dass** als Phosphin im katalytisch aktiven Palladium-Phosphin-Komplex ein Diphosphin der allgemeinen Formel
R⁴R⁵P―Q―PR⁶R⁷ IV,
eingesetzt wird, worin R⁴ bis R⁷ unabhängig voneinander Phenyl, substituiertes Phenyl, C₁₋₆-Alkyl oder C₃₋₆-Cycloalkyl bedeuten, und Q eine 1,1'-Ferrocendiylgruppe oder eine Gruppe der Formel -[CH₂]_{*n*}-, worin *n* 3 oder 4 ist, bedeutet.

7. Verfahren nach einem der Ansprüche 1 bis 6, **dadurch gekennzeichnet, dass** der katalytisch aktive Palladium-Phosphin-Komplex *in situ* aus dem Phosphin und Palladium(II)acetat oder Dichloro-bis(triphenylphosphin)palladium(II) gebildet wird.

8. Verfahren nach einem der Ansprüche 1 bis 7, **dadurch gekennzeichnet, dass** als Base eine Base aus der Gruppe bestehend aus den Alkali- und Erdalkalisalzen von niedrigen Carbonsäuren, den Alkali- und Erdalkalihydrogencarbonaten und den Alkali- und Erdalkali(di)hydrogenphosphaten eingesetzt wird.

9. Verfahren nach einem der Ansprüche 1 bis 8, **dadurch gekennzeichnet, dass** als Base ein Alkaliacetat, vorzugsweise Natriumacetat oder Kaliumacetat, eingesetzt wird.

## Claims

1. A process for the production of 2-pyrimidinecarboxylic acid esters of the general formula 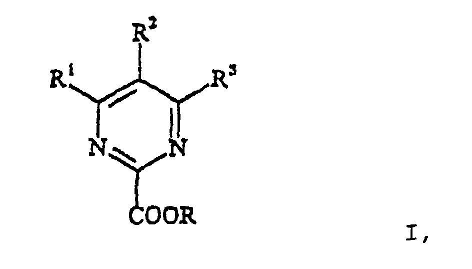 in which R means C₁₋₆ alkyl, C₃₋₆ cycloalkyl or aryl optionally substituted with C₁₋₆ alkyl, halogenated or unhalogenated, C₁₋₆ alkoxy, C₁₋₆ alkylthio, C₁₋₆ alkanesulfonyl or fluorine, or aryl-C₁₋₆-alkyl and R¹ to R³ mutually independently mean hydrogen, C₁₋₆ alkyl, fluorinated C₁₋₆ alkyl, C₁₋₆ alkoxy, (C₁₋₆-alkoxy) -C₁₋₆-alkyl or (C₁₋₆-alkoxy)carbonyl, **characterised in that** a 2-halopyrimidine of the general formula 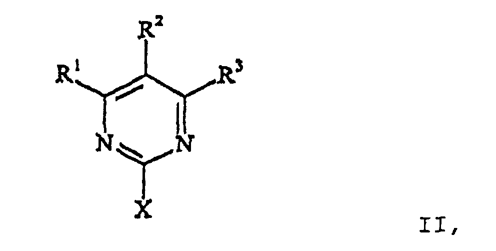 in which R¹ to R³ have the above-stated meanings and X is chlorine or bromine, is reacted with carbon monoxide and an alcohol of the general formula
R-OH III,
in which R has the above-stated meaning, in the presence of a catalytically active palladium-phosphine complex and a base.

2. A process according to claim 1, **characterised in that** X is chlorine.

3. A process according to claim 1 or 2, **characterised in that** R is C₁₋₄ alkyl.

4. A process according to one of claims 1 to 3, **characterised in that** R² is hydrogen.

5. A process according to one of claims 1 to 4, **characterised in that** R¹ and R³ are mutually independently hydrogen, C₁₋₄ alkoxy, (C₁₋₄-alkoxy)carbonyl or (C₁₋₄-alkoxy)methyl.

6. A process according to one of claims 1 to 5, **characterised in that** the phosphine used in the catalytically active palladium-phosphine complex is a diphosphine of the general formula
R⁴R⁵P―Q―PR⁶R⁷ IV,
in which R⁴ to R⁷ mutually independently mean phenyl, substituted phenyl, C₁₋₆ alkyl or C₃₋₆ cycloalkyl and Q means a 1,1'-ferrocenediyl group or a group of the formula -[CH₂]_{*n*}-, in which *n* is 3 or 4.

7. A process according to one of claims 1 to 6, **characterised in that** the catalytically active palladium-phosphine complex is formed *in situ* from the phosphine and palladium(II) acetate or dichloro-bis(triphenylphosphine)palladium(II).

8. A process according to one of claims 1 to 7, **characterised in that** the base used is a base from the group consisting of alkali metal and alkaline earth metal salts of low carboxylic acids, alkali metal and alkaline earth metal hydrogencarbonates and alkali metal and alkaline earth metal (di)hydrogenphosphates.

9. A process according to one of claims 1 to 8, **characterised in that** the base used is an alkali metal acetate, preferably sodium acetate or potassium acetate.

## Revendications

1. Procédé pour la préparation d'esters de l'acide pyrimidine-2-carboxylique de la formule générale 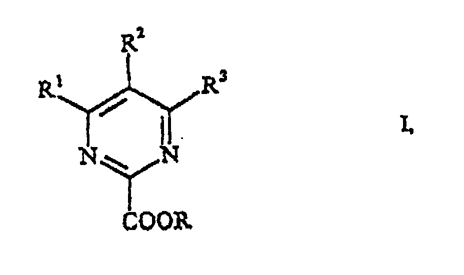 dans laquelle R est alkyle en C₁₋₆, cycloalkyle en C₃₋₆ ou éventuellement avec alkyle en C₁₋₆, halogéné ou non halogéné, alcoxy en C₁₋₆, alkylthio en C₁₋₆, alcanesulfonyle en C₁₋₆ ou aryle substitué par fluor ou alkyle en C₁₋₆-aryle et R¹ à R³ signifient indépendamment l'un de l'autre l'hydrogène, alkyle en C₁₋₆, alkyle en C₁₋₆ fluoré, alcoxy en C₁₋₆, alkyle en C₁₋₆-(alcoxy en C₁₋₆) ou carbonyle-(alcoxy en C₁₋₆), **caractérisé en ce qu'**une 2-halogénopyrimidine de la formule générale 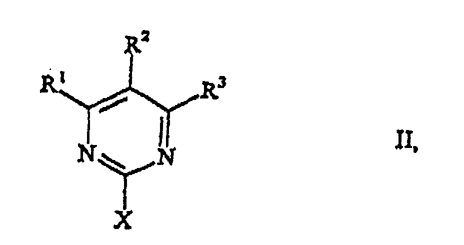 dans laquelle R¹ à R³ ont la signification précitée et X est le chlore ou le brome, est mise en réaction avec un monoxyde de carbone et un alcool de la formule générale
R-OH III,
dans laquelle R a la signification précitée, en présence d'un complexe palladium-phosphine catalytiquement actif et d'une base.

2. Procédé selon la revendication 1, **caractérisé en ce que** X est le chlore.

3. Procédé selon la revendication 1 ou 2, **caractérisé en ce que** R est alkyle en C₁₋₄.

4. Procédé selon l'une des revendications 1 à 3, **caractérisé en ce que** R² est de l'hydrogène.

5. Procédé selon l'une des revendications 1 à 4, **caractérisé en ce que** R¹ et R³ sont indépendamment l'un de l'autre de l'hydrogène, alcoxy en C₁₋₄, carbonyle(alcoxy en C₁₋₄), ou méthyle(alcoxy en C₁₋₄).

6. Procédé selon l'une des revendications 1 à 5, **caractérisé en ce qu'**en tant que phosphine dans le complexe palladium-phosphine catalytiquement actif on met en oeuvre une diphosphine de la formule générale
R⁴R⁵P―Q―PR⁶R⁷ IV,
dans laquelle R⁴ à R⁷ signifient indépendamment l'un de l'autre phényle, phényle substitué, alkyle en C₁₋₆ ou cycloalkyle en C₃₋₆, et Q est un groupe 1,1'-ferrocendiyle ou un groupe de la formule -[CH₂]ₙ-, dans lequel ₙ est 3 ou 4.

7. Procédé selon l'une des revendications 1 à 6, **caractérisé en ce que** le complexe palladium-phosphine catalytiquement actif est formé *in situ* à partir de phosphine et palladium(II)acétate ou de dichloro-bis(triphénylphosphine)palladium(II).

8. Procédé selon l'une des revendications 1 à 7, **caractérisé en ce qu'**en tant que base on met en oeuvre une base du groupe constitué par les sels alcalins et alcalino-terreux d'acides carboxyliques inférieurs, les hydrogénocarbonates alcalins et alcalino-terreux et les (di)hydrogénophosphates alcalins et alcalino-terreux.

9. Procédé selon l'une des revendications 1 à 8, **caractérisé en ce qu'**en tant que base on met en oeuvre un acétate alcalin, de préférence un acétate de sodium ou un acétate de potassium.
